(19) **Europäisches Patentamt / European Patent Office / Office européen des brevets**

(11) **EP 2 455 096 A1**

(12) **EUROPEAN PATENT APPLICATION**
published in accordance with Art. 153(4) EPC

(43) Date of publication:
**23.05.2012 Bulletin 2012/21**

(21) Application number: **10797044.4**

(22) Date of filing: **29.06.2010**

(51) Int Cl.:
*A61K 36/18* (2006.01)    *A61P 1/16* (2006.01)
*A61P 3/10* (2006.01)    *A61P 7/00* (2006.01)
*A61P 9/00* (2006.01)    *A61P 9/10* (2006.01)
*A61P 13/12* (2006.01)    *A61P 17/00* (2006.01)
*A61P 19/02* (2006.01)    *A61P 19/08* (2006.01)
*A61P 19/10* (2006.01)    *A61P 25/00* (2006.01)
*A61P 25/28* (2006.01)    *A61P 27/02* (2006.01)
*A61P 27/12* (2006.01)    *A61P 29/00* (2006.01)
*A61P 43/00* (2006.01)

(86) International application number:
**PCT/JP2010/061062**

(87) International publication number:
**WO 2011/004733 (13.01.2011 Gazette 2011/02)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **08.07.2009 JP 2009162188**

(71) Applicant: **Arkray, Inc.
Minami-ku
Kyoto-shi
Kyoto 601-8045 (JP)**

(72) Inventors:
• **YAGI, Masayuki.
Kyoto 602-0008 (JP)**
• **TAKAHASHI, Tomoko
Fukushima 963- 8611 (JP)**

(74) Representative: **Jones, Elizabeth Louise
Dehns
St Bride's House
10 Salisbury Square
GB-London EC4Y 8JD (GB)**

(54) **OXIDIZED PROTEIN HYDROLASE ACTIVITY ENHANCER**

(57) The present invention provides an enhancer for enhancing the activity of oxidized protein hydrolase. The oxidized protein hydrolase activity enhancer according to the present invention contains an extract of at least one plant selected from the group consisting of *Compositae Anthemis, Saururaceae Houttuynia, Rosaceae Crataegus,* and *Vitaceae Vitis.* The oxidized protein hydrolase activity enhancer preferably contains an extract of a plant of *Compositae Anthemis,* an extract of a plant of *Saururaceae Houttuynia,* an extract of a plant of *Rosaceae Crataegus,* and an extract of a plant of *Vitaceae Vitis.* It is preferable that the plant of *Compositae Anthemis* is *Anthemis nobilis L.,* the plant of *Saururaceae Houttuynia* is *Houttuynia cordata Thunberg,* the plant of *Rosaceae Crataegus* is *Crataegus oxyacantha L.,* and the plant of *Vitaceae Vitis* is *Vitis vinifera L.*

FIG. 1

**Description**

Technical Field

The present invention relates to an enhancer for enhancing the activity of oxidized protein hydrolase.

Background Art

It is known that oxidation reactions and glycation reactions *in vivo* adversely affect cells and tissues. In particular, the production and accumulation of proteins having undergone an oxidation reaction or a glycation reaction, i.e., oxidized proteins or glycated proteins, cause diseases such as diabetes complications, Alzheimer's disease, cataracts, and arteriosclerosis, as well as senescence and hypofunction of tissues such as skin (Non-Patent Document 1). On account of this, oxidized proteins and glycated proteins are referred to as "damaged proteins".

Although these damaged proteins normally are degraded and removed by proteases such as proteasomes and oxidized protein hydrolase (hereinafter referred to as "OPH"), the activities of these enzymes are reduced with age (Non-Patent Document 2). Expectations are rising regarding the possibility of treating and preventing diseases, the senescence of tissues, and the hypofunction of tissues by enhancing the activities of these enzymes.

Proteasomes are large enzyme complexes that degrade proteins, with examples thereof including 26S proteasome and 20S proteasome. Known substances that activate proteasomes are soybean saponin, kale extract, silybin, silane compounds, iris extract, and the like (Patent Documents 1 to 5). On the other hand, OPH is a serine protease and is the same as acylpeptide hydrolase (ACPH) that releases N-acylated amino acids in proteins (Non-Patent Document 3). It is also called acylamino-acid-releasing enzyme (AARE). OPH is present in human erythrocytes and various other tissues such as liver, kidney, brain, and heart, and preferentially degrades oxidized proteins and glycated proteins (Non-Patent Document 4). It has been reported that a marked increase in OPH activity in serum, for example, is seen in diabetic rats, and carbonylated proteins (oxidized proteins) that accumulate in the serum of the diabetic rats are degraded owing to the increase in the OPH activity (Non-Patent Document 5). As described above, since OPH is closely involved in the above-described diseases and the like, there is a demand for enhancement of OPH activity.

Citation List

[Patent Documents]

    [Patent Document 1] JP 2002-179592 A

[Patent Document 2] Japanese Patent No. 4255432
[Patent Document 3] JP 2008-308505 A
[Patent Document 4] JP 2004-91398 A
[Patent Document 5] JP 2007-99650 A

[Non-Patent Documents]

    [Non-Patent Document 1] Goto et al., Experimental Medicine, 18 (special number), pp. 2324-2331, 1998
    [Non-Patent Document 2] Breusing N. et al., Biol. Chem., Vol. 389, pp. 203-209 (2008)
    [Non-Patent Document 3] Fujino et al., Biochim. Biophys. Acta, Vol. 1478, pp. 102-112 (2000)
    [Non-Patent Document 4] Fujino et al., J. Biochem., Vol. 124, pp. 1077-1085 (1998)
    [Non-Patent Document 5] Shimizu et al., Biol. Pharm. Bull. Vol. 32(9) in press (2009)

Disclosure of Invention

Problem to be Solved by the Invention

An object of the present invention is to provide an OPH activity enhancer.

Means for Solving Problem

The present invention provides an OPH activity enhancer containing an extract of at least one plant selected from the group consisting of

*Compositae Anthemis, Saururaceae Houttuynia, Rosaceae Crataegus,* and *Vitaceae Vitis.*

The present invention also provides a method for enhancing OPH activity, including adding the OPH activity enhancer of the present invention to a sample containing OPH.

The present invention also provides a method for accelerating degradation of a protein, including adding the OPH activity enhancer of the present invention to a sample containing a protein and OPH. The protein includes at least one of an oxidized protein and a glycated protein.

The present invention also provides a method for treating diseases, senescence of tissues, and hypofunction of tissues caused by production of an oxidized protein or a glycated protein, including administering the OPH activity enhancer of the present invention to a living organism.

Effects of the Invention

According to the present invention, it is possible to enhance OPH activity. Thus, the present invention can accelerate the degradation of damaged proteins such as oxidized proteins and glycated proteins, and can treat and prevent diseases, senescence of tissues, and hypofunction of tissues caused by the damaged proteins produced *in vivo,* for example. Furthermore, the present invention is excellent in terms of safety because it uses plant extracts that have been used since ancient times. Moreover, the present invention is excellent in terms of productivity because the plant extracts can be obtained in large quantities.

Brief Description of Drawings

[FIG. 1] FIG. 1 is a graph showing the change in absorbance, which indicates OPH activity, with time when respective plant extracts, a mixed extract thereof, catechin, and a control were used.
[FIG. 2] FIG. 2 is a graph showing the OPH activity increase/decrease rate (%) relative to the control at the reaction time of 100 minutes when the respective plant extracts, the mixed extract thereof, and the catechin were used.

Mode for Carrying out the Invention

In the present invention, enhancement of the activity of an enzyme may be activation of the enzyme or inhibition of deactivation of the enzyme, for example. In the present invention, OPH is also referred to as acylamino-acid-releasing enzyme (AARE) or acylpeptide hydrolase (ACPH).

The OPH activity enhancer of the present invention contains an extract of at least one plant selected from the group consisting of *Compositae Anthemis, Saururaceae Houttuynia, Rosaceae Crataegus,* and *Vitaceae Vitis,* as described above. The OPH activity enhancer may contain an extract of one of the above-described plants, or it may contain extracts of two or more of the above-described plants, for example. The OPH activity enhancer may contain: at least one of an extract of a plant of *Compositae Anthemis* and an extract of a plant of *Saururaceae Houttuynia;* and at least one of an extract of a plant of *Rosaceae Crataegus* and an extract of a plant of *Vitaceae Vitis,* for example.

The OPH activity enhancer preferably contains: an extract of a plant of *Compositae Anthemis;* an extract of a plant of *Saururaceae Houttuynia;* an extract of a plant of *Rosaceae Crataegus*; and an extract of a plant of *Vitaceae Vitis,* for example. It is speculated that, among these extracts, the extract of the plant of *Compositae Anthemis* and the extract of the plant of *Saururaceae Houttuynia* activate OPH, for example, and the extract of the plant of *Rosaceae Crataegus* and the extract of the plant of *Vitaceae Vitis* inhibit the deactivation of OPH, for example. This speculation, however, by no means limits the present invention.

When the OPH activity enhancer contains an extract (A) of a plant of *Compositae Anthemis,* an extract (B) of a plant of *Saururaceae Houttuynia,* an extract (C) of a plant of *Rosaceae Crataegus,* and an extract (D) of a plant of *Vitaceae Vitis,* the blending ratio of these extracts (the dry weight ratio, A : B : C : D) is not particularly limited, and preferably is 1 : 0.01 to 100 : 0.01 to 100 : 0.01 to 100, for example.

Examples of the plant of *Compositae Anthemis* (*Chamaemelum*) include *Anthemis nobilis L.* (= *Chamaemelum nobile*). An extract of the plant of *Compositae Anthemis* may be obtained from its flower, spike, pericarp, fruit, stem, leaf, rhizome, root bark, root, and seed, for example. The extract may be obtained from one part or from at least two parts. Alternatively, the extract may be obtained from the entire plant. In the extract of the plant of *Compositae Anthemis,* the part from which the extract is obtained is not particularly limited, and the extract may be an extract of anthodia, for example.

Examples of the plant of *Saururaceae Houttuynia* include *Houttuynia cordata Thunberg.* An extract of the plant of *Saururaceae Houttuynia* may be obtained from its flower, spike, pericarp, fruit, stem, leaf, rhizome, root bark, root, and seed, for example. The extract may be obtained from one part or from at least two parts. Alternatively, the extract may be obtained from the entire plant. In the extract of the plant of *Saururaceae Houttuynia,* the part from which the extract is obtained is not particularly limited, and the extract may be an extract of aerial parts such as its flower, spike, pericarp, fruit, stem, leaf, branch, branches and leaves, trunk, bark, and seed for example.

Examples of the plant of *Rosaceae Crataegus* include *Crataegus oxyacantha L.* and *C. cuneata Sieb. et Zucc.* An extract of the plant of *Rosaceae Crataegus* may be obtained from its flower, spike, pericarp, fruit, stem, leaf, branch, branches and leaves, trunk, bark, rhizome, root bark, root, and seed, for example. The extract may be obtained from one part or from at least two parts. Alternatively, the extract may be obtained from the entire plant. In the extract of the plant of *Rosaceae Crataegus,* the part from which the extract is obtained is not particularly limited, and the extract may be an extract of fruits, for example.

Examples of the plant of the *Vitaceae Vitis* include: *Vitis vinifera L.*; *Vitis labrusca L.*; *V. saccharifera Makino*; *V. ficifolia Bunge var. lobata* (*Regel*) *Nakai*; *V. flexuosa Thunb.*; *V. coiguetiae Pulliat*; and *V. labruscana Bailey.* An extract of the plant of *Vitaceae Vitis* may be obtained from its flower, spike, pericarp, fruit, stem, leaf, branch, branches and leaves, trunk, bark, rhizome, root bark, root, and seed, for example. The extract may be obtained from one part or from at least two parts. Alternatively, the extract may be obtained from the entire plant. In the extract of the plant of *Vitaceae Vitis,* the part from which the extract is obtained is not particularly limited, and the extract may be an extract of leaves, for example.

The OPH activity enhancer of the present invention preferably contains an extract of at least one plant selected from the group consisting of *Anthemis nobilis L., Houttuynia cordata Thunberg, Crataegus oxyacantha L.,* and *Vitis vinifera L.,* for example. The OPH activity enhancer may contain an extract of one of the above-described plants, or may contain extracts of two or more of the above-described plants, for example. The OPH activity enhancer may contain: at least one of an extract of *Anthemis nobilis L.* and an extract of *Houttuynia cordata Thunberg,* and at least one of an extract of *Crataegus oxyacantha L.* and an extract of *Vitis vinifera L.,* for example.

The OPH activity enhancer preferably contains an extract of *Anthemis nobilis L.,* an extract of *Houttuynia cordata Thunberg,* an extract of *Crataegus oxyacantha L.,* and an extract of *Vitis vinifera L.,* for example. It is speculated that, among these extracts, the extract of *Anthemis nobilis L.* and the extract of *Houttuynia cordata Thunberg* activate OPH, for example, and the extract of *Crataegus oxyacantha L.* and the extract of *Vitis vinifera L.* inhibit the deactivation of OPH, for example. This speculation, however, by no means limits the present invention.

When the OPH activity enhancer contains an extract (a) of *Anthemis nobilis L.,* an extract (b) of *Houttuynia cordata Thunberg,* an extract (c) of *Crataegus oxyacantha L.,* and an extract (d) of *Vitis vinifera L.,* the blending ratio of these extracts (the dry weight ratio, a : b : c : d) is not particularly limited, and preferably is 1 : 0.01 to 100 : 0.01 to 100 : 0.01 to 100, for example.

The OPH activity enhancer of the present invention preferably contains at least one selected from the group consisting of an extract obtained from the anthodia of a plant of *Compositae Anthemis,* an extract obtained from the aerial parts of a plant of *Saururaceae Houttuynia,* an extract obtained from the fruits of a plant of *Rosaceae Crataegus,* and an extract obtained from the leaves of a plant of *Vitaceae Vitis,* for example. The OPH activity enhancer may contain one kind, two or more kinds, or all kinds of the above-described extracts, for example. Specifically, the OPH activity enhancer preferably contains, for example, at least one selected from the group consisting of an extract obtained from the anthodia of *Anthemis nobilis L.*, an extract obtained from the aerial parts of *Houttuynia cordata Thunberg,* an extract obtained from the fruits of *Crataegus oxyacantha L.,* and an extract obtained from the leaves of *Vitis vinifera L.* The OPH activity enhancer may contain one kind, two or more kinds, or all kinds of the above-described extracts, for example.

In the present invention, the extract can be obtained from any of the above-described parts or from the entire plant, for example. The method for obtaining the extract is not particularly limited, and examples thereof include known methods such as solvent extraction and expression.

An extraction solvent to be used in the solvent extraction is not particularly limited, and examples thereof include aqueous solvents and organic solvents. The aqueous solvents are not particularly limited, and examples thereof include water. The organic solvents are not particularly limited, and examples thereof include lower alcohols, polyhydric alcohols, ketones, esters, ethers, nitriles, aromatic compounds, and alkyl chlorides. The lower alcohols are not particularly limited, and examples thereof include methanol, ethanol, and absolute ethanol. The polyhydric alcohols are not particularly limited, and examples thereof include propylene glycol and 1,3-butylene glycol. The ketones are not particularly limited, and examples thereof include acetone and formic acid. The esters are not particularly limited, and examples thereof include ethyl acetate. The ethers are not particularly limited, and examples thereof include diethyl ether and dioxane. The nitriles are not particularly limited, and examples thereof include acetonitrile. The aromatic compounds are not particularly limited, and examples thereof include benzene, toluene, and xylene. The alkyl chlorides are not particularly limited, and examples thereof include chloroform.

As the above-described extraction solvent, one kind of solvent may be used, or two or more kinds of solvents may be used in combination, for example. Furthermore, the extraction solvent may be a mixed solvent of the aqueous solvent and the organic solvent, for example. The mixed solvent is not particularly limited, and examples thereof include lower alcohol aqueous solutions such as ethanol aqueous solutions. The proportion of the organic solvent in the mixed solvent is not particularly limited, and is, for example, 1 to 99 vol%.

The solvent extraction can be carried out by, for example, using any of the above-described parts or the entire plant as a raw material and immersing it in the extraction solvent. The raw material may be subjected to treatments such as, for example, washing, drying, and pulverizing, prior to the immersion. When there are two or more kinds of raw materials, each of the raw materials may be subjected to an extraction treatment separately, or a mixture of the two or more kinds of raw materials may be subjected to an extraction treatment. In the former case, for example, the resultant extracts may be mixed together to provide a mixed extract. The proportions of the respective extracts in the mixed extract are not particularly limited, and may be equal proportions (weights), for example. For example, in the case where the above-described four kinds of plants are used, a mixed extract preferably is prepared by mixing extracts of these plants at a ratio (weight ratio) of 1 : 1 : 1 : 1. In the latter case, for example, the proportions of the respective raw materials in the mixture are not particularly limited, and may be equal proportions (weights), for example. For example, when the above-described four kinds of plants are used, it is preferable to perform an extraction treatment with respect to a mixture obtained by mixing the respective raw materials at a ratio (weight ratio) of 1 : 1 : 1 : 1.

The ratio between the raw material used in the extraction and the extraction solvent is not particularly limited, and may be, for example, 100 g (dry weight) of the raw material and 1 to 1000 1 of the extraction solvent, preferably 1 to 100 1 of the extraction solvent. The immersion time can be set as appropriate depending on, for example, the kinds, the amounts, and the like of the raw material and the extraction solvent, and is not particularly limited. Specifically, in the case where 100 g of the raw material is immersed in 10 1 of the extraction solvent, the immersion time preferably is 0.5 hours or more, more preferably 0.5 to 24 hours, for example.

The temperature of the extraction solvent at the time of extraction is not particularly limited, and may be room temperature, equal to or higher than room temperature, or equal to or lower than room temperature. When the extraction solvent is an aqueous solvent, the extraction treatment preferably is hot water extraction, for example. In the case of hot water extraction, the temperature of the aqueous solvent is not particularly limited, and may be, for example, 30˚C or higher, preferably 50˚C to 100˚C. Furthermore, the treatment time in the hot water extraction can be set as appropriate depending on, for example, the kind and the amount of the raw material, the amount of the aqueous solvent, and the like, and is not particularly limited. Specifically, when extraction from 100 g (dry weight) of the raw material is performed using 101 of the aqueous solvent, the treatment time preferably is 0.5 hours or longer, more preferably 0.5 to 24 hours.

After the extraction treatment, the extract obtained may be subjected to a purification treatment, for example. The purification treatment is not particularly limited, and examples thereof include known methods such as a distillation treatment, a filtration treatment, a chromatography treatment, and a drying treatment.

The form of the extract is not particularly limited. For example, the extract may be in the form of liquid, paste, powder, or the like. The form of the extract can be selected as appropriate depending on the form of the OPH activity enhancer described below.

The OPH activity enhancer may contain only the above-described extract, or it may be a composition containing the above-described extract and other components. Examples of the components include various kinds of additives such as an excipient, a binding agent, a lubricant, a disintegrant, an absorption promoter, an emulsifying agent, a stabilizing agent, and an antiseptic agent. The blended amounts of the various kinds of additives in the OPH activity enhancer are not particularly limited, and can be set as appropriate.

The form of the OPH activity enhancer is not particularly limited. For example, the OPH activity enhancer may be in the form of solid, gel, liquid, or the like. Particular examples of the form of the OPH activity enhancer include powder, microgranule, granule, tablet, coated tablet, capsule, troche, eye drop, liquid, patch, lotion, and ointment.

The OPH activity *in vivo* can be enhanced by administering the OPH activity enhancer of the present invention to a living organism, for example. Thus, the OPH activity enhancer can be used in, for example, a method for treating or preventing diseases, senescence of tissues, hypofunction of tissues, and the like caused by an oxidized protein or a glycated protein *in vivo,* as will be described below. The OPH activity enhancer may be administered in the form of a composition obtained by mixing it with components of another article, e.g., foods such as common foods and nutritional supplements, cosmetics, quasi drugs, and pharmaceuticals, for example. The blended amount of the OPH activity enhancer in the composition can be set as appropriate depending on, for example, the kind of the article, subjects to which the composition is administered, and the like, and is not particularly limited.

The method for enhancing the OPH activity according to the present invention includes adding the OPH activity enhancer of the present invention to a sample containing OPH, as described above. The method for enhancing the OPH activity is applicable to methods that include releasing an N-acylated amino acid, for example. Specifically, the method for enhancing the OPH activity can be used in a protein sequence analysis, for example. In the protein sequence analysis, by, for example, releasing an N-acylated amino acid in an analysis target protein with OPH in the presence of the OPH activity enhancer, it is possible to release the N-acylated amino acid more efficiently. The protein sequence analysis is not particularly limited as long as it uses the method for enhancing OPH activity. Any conventionally known method can be employed. In the releasing step, the amount of the added OPH activity enhancer in the reaction solution can be set as appropriate depending on, for example, the amount of the analysis target protein, and is not particularly limited.

An exemplary method for accelerating degradation of a protein according to the present invention includes adding the OPH activity enhancer of the present invention to a sample containing a protein and OPH, where the protein includes at least one of an oxidized protein and a glycated protein, as described above. This method for accelerating degradation of a protein is applicable to methods that include releasing an N-acylated amino acid, for example. Specifically, the method for accelerating degradation of a protein can be used in the above-described protein sequence analysis, for example.

Examples of the oxidized protein and glycated protein include: proteins modified by diseases showing pathological conditions due to aging or senescence; proteins suffering oxidative damage by, for example, exposure to ultraviolet rays; and proteins modified by sugar. Examples of the modification of the proteins include oxidation, glycation, carbonylation, and acylation. The carbonylation encompasses conversion to hydroxynonenal, malondialdehyde, glycolaldehyde, and pyrraline, for example. Specifically, examples of the oxidized protein include, but are not limited to, oxidized oxiredoxin, and examples of the glycated protein include, but are not limited to, HbA1c, glycoalbumin, and proteins in the form of AGEs (advanced glycation end products).

The treatment method according to the present invention treats diseases, senescence of tissues, and/or hypofunction of tissues caused by the production of an oxidized protein or a glycated protein by administering the OPH activity enhancer of the present invention to a living organism. The treatment method of the present invention may be a prevention method. Examples of the diseases include diabetes complications, Alzheimer's disease, cataracts, arteriosclerosis, osteoporosis, rheumatism, and osteoarthropathy. Examples of the tissues include skin, nerve, kidney, blood vessel, retina, crystalline lens, bone, joint, brain tissue, liver, and erythrocyte. The species of the living organism is not particularly limited, with examples thereof including: humans; non-human mammals such as monkeys, cows, pigs, dogs, and cats; birds such as chickens; and fish and shellfish. The administration method is not particularly limited, with examples thereof including oral administration and parenteral administration. Examples of the parenteral administration include transdermal administration and injections such as intravenous injection. The form of the OPH activity enhancer is not particularly limited, and may be in any of the above-described forms, for example. Furthermore, the OPH activity enhancer may be administered in the form of a composition obtained by mixing it with other components, as described above. The blended amount of the OPH activity enhancer in the composition can be set as appropriate, and is not particularly limited, as described above. The dose of the OPH activity enhancer can be set as appropriate depending on, for example, the animal species and age, and is not particularly limited. When a healthy adult orally takes the OPH activity enhancer, the amount of the extract (solid content) taken preferably is 10 to 2000 mg, more preferably 50 to 1000 mg , for example. The OPH activity enhancer of the present invention also can be referred to as a therapeutic agent for treating the above-described diseases, senescence of tissues, and/or hypofunction of tissues, and it also encompasses a prophylactic agent, for example.

An extract of the present invention is an extract of a plant of *Compositae Anthemis, Saururaceae Houttuynia, Rosaceae Crataegus,* or *Vitaceae Vitis,* for use in the treatment of diseases, senescence of tissues, and/or hypofunction of tissues caused by the production of an oxidized protein or a glycated protein. The treatment may be prevention. A composition of the present invention is a composition containing an extract of at least one plant selected from the group consisting of plants of *Compositae Anthemis, Saururaceae Houttuynia, Rosaceae Crataegus,* and *Vitaceae Vitis,* for use in the treatment of diseases, senescence of tissues, and/or hypofunction of tissues caused by the production of an oxidized protein or a glycated protein. The treatment may be prevention. In the extract and the composition of the present invention, the extract of each kind and the composition containing the extract are the same as described above, and the combination, the method for using them, and the like also are the same as described above. Examples

Next, examples of the present invention will be described. It is to be noted, however, the present invention is by no means limited by the following examples.

In the present examples, the influence of plant extracts and a mixed extract on the OPH activity was evaluated. Plants used in the present examples were: anthodia of *Anthemis nobilis L.,* aerial parts of *Houttuynia cordata Thunberg,* fruits of *Crataegus oxyacantha L.,* and leaves of *Vitis vinifera L*. As the OPH, a commercially available product AARE (TAKARA BIO INC.) was used.

Preparation of Plant Extract

100 g of each of the plants in a dried state was immersed in 10 1 of purified water at 80°C for about 5 hours, whereby a liquid extract of each plant was obtained. Each liquid extract was filtered to remove the residue, and about 10 kg of the filtrate was collected. Each filtrate was further dried to remove the solvent. Thus, extracts of the respective plants in the form of powder were obtained. Furthermore, as the mixed extract, "AG Herb MIX" (trade name, ARKRAY, Inc.) was used. The plant extracts and the mixed extract were each dissolved in water so that their concentrations were 35 mg/ml. The mixtures thus obtained were used as samples.

Evaluation of OPH activity

As examples, 495 μl of reaction solutions containing the samples respectively and having the following composition were prepared. As a control, a reaction solution having the same composition but containing 5 μl of water instead of the sample was prepared. As a reference example, a reaction solution was prepared by adding catechin (trade name "Polyphenon 100", Kurita Water Industries Ltd.) as an antioxidant instead of the sample. Specifically, the reaction solution was prepared in the same manner except that 5 μl of 3.5 mg/ml catechin was added instead of the sample.

(Reaction Solution)

| Component | Blended amount (μl) |
|---|---|
| 100 mmol/l Tris-HCl (pH 7.4) | 485 |
| 0.1 U/ml AARE solution | 5 |
| 35 mg/ml sample | 5 |

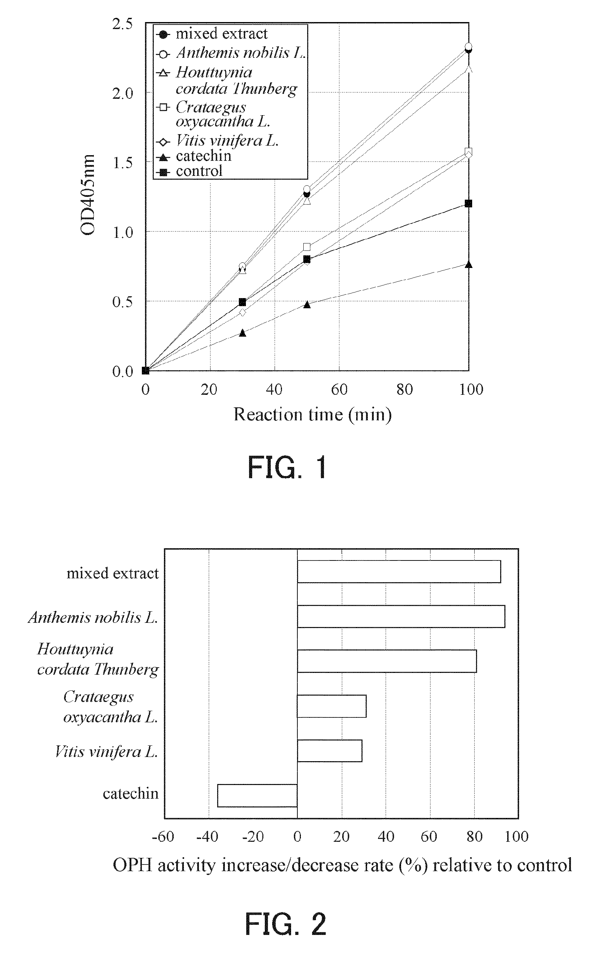

495 μl of each of the reaction solutions was allowed to stand still at room temperature for 20 minutes. 5 μl of 200 mmol/l AAPA (N-acetyl-L-alanine-p-nitroanilide) solution (Calbiochem) was further added thereto as a substrate, and the resultant mixture was allowed to react at 37˚C for 100 minutes. Then, after a lapse of predetermined times from the start of the reaction (i.e., at the reaction times of 0, 30, 50, and 100 minutes), the absorbance of the reaction solution was measured at a measurement wavelength of 405 nm using a microplate reader. A higher absorbance in the reaction solution of the example compared to the control means that the reaction solution has higher enzyme activity. The results thereof are shown in FIG. 1. FIG. 1 is a graph showing change in absorbance indicating OPH activity with time when the respective plant extracts, the mixed extract thereof, the catechin, and the control were used. In FIG. 1, the horizontal axis indicates a reaction time (minutes), and the vertical axis indicates an absorbance at a measurement wavelength of 405 nm. In the graph of FIG. 1, an open circle (○) indicates the measurement result regarding the extract of *Anthemis nobilis L.,* an open triangle (△) indicates the measurement result regarding the extract of *Houttuynia cordata Thunberg,* an open square (□) indicates the measurement result regarding the extract of *Crataegus oxyacantha L.,* an open rhombus (◇) indicates the measurement result regarding the extract of *Vitis vinifera L.,* a filled circle (•) indicates the measurement result regarding the mixed extract, a filled triangle (▲) indicates the measurement result regarding the reference example (catechin), and a filled square (■) indicates the measurement result regarding the control. Furthermore, regarding the absorbance at the reaction time of 100 minutes, the OPH activity increase/decrease rate (R, %) relative to the control was determined based on the following equation.

$$R = [(a - b)/b] \times 100$$

a: the absorbance of the reaction solution of the example or the reference example
b: the absorbance of the control

The results thereof are shown in FIG. 2. FIG. 2 is a graph showing the OPH activity increase/decrease rate (%) relative to the control at the reaction time of 100 minutes when the respective plant extracts, the mixed extract thereof, and the catechin were used. In FIG. 2, the horizontal axis indicates the OPH activity increase/ decrease rate (%) relative to the control. The results shown are, from the top, the results regarding the mixed extract, the extract of *Anthemis nobilis L.,* the extract of *Houttuynia cordata Thunberg,* the extract of *Crataegus oxyacantha L.,* the extract of *Vitis vinifera L.,* and the catechin.

As can be seen from FIGs. 1 and 2, the reactivity of the control decreased after the reaction time of 50 minutes. In contrast, in all the examples using the extract of *Anthemis nobilis L.,* the extract of *Houttuynia cordata Thunberg,* the extract of *Crataegus oxyacantha L.,* the extract of *Vitis vinifera L.* and the mixed extract, respectively, absorbances higher than those of the control and the reference example were observed at the reaction time of 100 minutes. From these results, it was found that the OPH activity was enhanced. In particular, in the case where the extract of *Anthemis nobilis L.,* the extract of *Houttuynia cordata Thunberg,* and the mixed extract were used, absorbances higher than that of the control were observed at the reaction time of 50 minutes. These results demonstrate that the OPH activity can be enhanced by the respective plant extracts and the mixed extract thereof. Therefore, it can be said that they can accelerate the degradation and removal of oxidized proteins and glycated proteins.

Industrial Applicability

According to the present invention, it is possible to enhance OPH activity. Therefore, the present invention can be used to accelerate the degradation of damaged proteins such as oxidized proteins and glycated proteins, so that it can be used to treat or prevent diseases, senescence of tissues, and hypofunction of tissues caused by the damaged proteins generated *in vivo*. The present invention shows excellent safety because it uses plant extracts that have been used since ancient times. Also, the present invention shows excellent productivity because the plant extracts can be obtained in large quantities. The present invention is applicable to a wide range of fields such as industrial products, foods, and pharmaceuticals.

**Claims**

1. An oxidized protein hydrolase activity enhancer comprising:

   an extract of at least one plant selected from the group consisting of *Compositae Anthemis, Saururaceae Houttuynia, Rosaceae Crataegus,* and *Vitaceae Vitis.*

2. The oxidized protein hydrolase activity enhancer according to claim 1, comprising:

   an extract of a plant of *Compositae Anthemis*;
   an extract of a plant of *Saururaceae Houttuynia*;
   an extract of a plant of *Rosaceae Crataegus*; and
   an extract of a plant of *Vitaceae Vitis.*

3. The oxidized protein hydrolase activity enhancer according to claim 1 or 2, comprising:

   at least one of an extract of a plant of *Compositae Anthemis* and an extract of a plant of *Saururaceae Houttuynia;* and

at least one of an extract of a plant of *Rosaceae Crataegus* and an extract of a plant of *Vitaceae Vitis.*

4. The oxidized protein hydrolase activity enhancer according to any one of claims 1 to 3, wherein
the plant of *Compositae Anthemis* is *Anthemis nobilis L.,*
the plant of *Saururaceae Houttuynia* is *Houttuynia cordata Thunberg,*
the plant of *Rosaceae Crataegus* is *Crataegus oxyacantha L.,* and the plant of *Vitaceae Vitis* is *Vitis vinifera L.*

5. A method for enhancing the activity of oxidized protein hydrolase, the method comprising:

adding the oxidized protein hydrolase activity enhancer according to any one of claims 1 to 4 to a sample comprising oxidized protein hydrolase.

6. A method for accelerating degradation of a protein, the method comprising:

adding the oxidized protein hydrolase activity enhancer according to any one of claims 1 to 4 to a sample comprising a protein and oxidized protein hydrolase,
wherein the protein comprises at least one of an oxidized protein and a glycated protein.

7. A method for treating diseases, senescence of tissues, and hypofunction of tissues caused by production of an oxidized protein or a glycated protein, the method comprising:

administering the oxidized protein hydrolase activity enhancer according to any one of claims 1 to 4 to a living organism.

FIG. 1

OPH activity increase/decrease rate (%) relative to control

FIG. 2

<table>
<tr><td colspan="2" align="center">**INTERNATIONAL SEARCH REPORT**</td><td>International application No.<br>PCT/JP2010/061062</td></tr>
</table>

| A. CLASSIFICATION OF SUBJECT MATTER |
|---|
| See extra sheet. |
| According to International Patent Classification (IPC) or to both national classification and IPC |

| B. FIELDS SEARCHED |
|---|
| Minimum documentation searched (classification system followed by classification symbols)<br>A61K36/18, A61P1/16, A61P3/10, A61P7/00, A61P9/00, A61P9/10, A61P13/12, A61P17/00, A61P19/02, A61P19/08, A61P19/10, A61P25/00, A61P25/28, A61P27/02, A61P27/12, A61P29/00, A61P43/00 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

CA/MEDLINE/EMBASE/BIOSIS(STN), JSTPlus/JMEDPlus/JST7580(JDreamII)

| C. DOCUMENTS CONSIDERED TO BE RELEVANT | | |
|---|---|---|
| Category* | Citation of document, with indication, where appropriate, of the relevant passages | Relevant to claim No. |
| X<br>Y | JP 2005-035911 A (Arkray, Inc.),<br>10 February 2005 (10.02.2005),<br>paragraphs [0002], [0004], [0037] to [0054]<br>(Family: none) | 1-6<br>1-6 |
| X<br>Y | JP 2008-231031 A (Arkray, Inc.),<br>02 October 2008 (02.10.2008),<br>paragraphs [0003], [0025] to [0030]<br>& WO 2008/0114536 A1 & US 2010/009016 A1 | 1-6<br>1-6 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>27 July, 2010 (27.07.10) | Date of mailing of the international search report<br>03 August, 2010 (03.08.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/061062

Continuation of A. CLASSIFICATION OF SUBJECT MATTER
  (International Patent Classification (IPC))

*A61K36/18*(2006.01)i, *A61P1/16*(2006.01)i, *A61P3/10*(2006.01)i,
*A61P7/00*(2006.01)i, *A61P9/00*(2006.01)i, *A61P9/10*(2006.01)i,
*A61P13/12*(2006.01)i, *A61P17/00*(2006.01)i, *A61P19/02*(2006.01)i,
*A61P19/08*(2006.01)i, *A61P19/10*(2006.01)i, *A61P25/00*(2006.01)i,
*A61P25/28*(2006.01)i, *A61P27/02*(2006.01)i, *A61P27/12*(2006.01)i,
*A61P29/00*(2006.01)i, *A61P43/00*(2006.01)i

        (According to International Patent Classification (IPC) or to both national
        classification and IPC)

Form PCT/ISA/210 (extra sheet) (July 2009)

## INTERNATIONAL SEARCH REPORT

| International application No. |
|---|
| PCT/JP2010/061062 |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☒ Claims Nos.: 7
   because they relate to subject matter not required to be searched by this Authority, namely:
   ```
   Claim 7 involves a "method for treatment of the human body or animal body
   by surgery or therapy".
   ```

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| **Box No. III** | **Observations where unity of invention is lacking (Continuation of item 3 of first sheet)** |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002179592 A
- JP 4255432 B
- JP 2008308505 A
- JP 2004091398 A
- JP 2007099650 A

**Non-patent literature cited in the description**

- **Goto et al.** *Experimental Medicine,* 1998, vol. 18, 2324-2331
- **Breusing N. et al.** *Biol. Chem.,* 2008, vol. 389, 203-209
- **Fujino et al.** *Biochim. Biophys. Acta,* 2000, vol. 1478, 102-112
- **Fujino et al.** *J. Biochem.,* 1998, vol. 124, 1077-1085
- **Shimizu et al.** *Biol. Pharm. Bull.,* 2009, vol. 32 (9